# FASCICULE DE BREVET EUROPEEN

(11) **EP 3 241 623 B1**
(45) Date de publication et mention de la délivrance du brevet: **14.08.2019**
(21) Numéro de dépôt: 17168034.1
(22) Date de dépôt: 25.04.2017
(51) Int. Cl.: B08B 3/12, A61C 17/00, F26B 3/02, B08B 3/04

(54) **CASSETTE DE STOCKAGE POUR PROTHÈSES DENTAIRES**
AUFBEWAHRUNGSBEHÄLTER FÜR ZAHNPROTHESEN
STORAGE CASSETTE FOR DENTAL PROSTHESES

(30) Priorité: 26.04.2016 FR 1653691; 12.04.2017 FR 1753187
(43) Date de publication de la demande: 08.11.2017
(73) Titulaire: Maurand, Philippe, 69007 Lyon (FR)
(72) Inventeur: Maurand, Philippe, 69007 Lyon (FR)
(74) Mandataire: Chevalier, Renaud Philippe

(56) Documents cités:
- WO-A1-2015/150986
- US-A- 2 122 583
- US-A- 3 640 295
- US-A- 4 409 999
- US-A- 5 378 287
- US-A- 5 482 067
- US-A1- 2007 104 609

## Description

La présente invention concerne le domaine des prothèses dentaires et plus spécifiquement du nettoyage des prothèses dentaires.

En maison de retraite, dans les structures de soins médicalisées, ou tout autre établissement collectif analogue, les prothèses dentaires sont généralement nettoyées par trempage dans des bains à ultrasons contenant une solution nettoyante pour éliminer la calcification et certaines bactéries présentes sur les prothèses dentaires.

Ce type de nettoyage présente l'inconvénient de laisser les prothèses dentaires humides et donc présentant un risque de contamination pathogène au contact de l'eau.

En outre, comme il s'agit d'un dispositif de nettoyage collectif, l'ensemble des prothèses dentaires sont recueillies puis disposées en vrac dans le bain afin de les nettoyer plus rapidement mais ce qui ralenti considérablement l'identification, le transport et la restitution des prothèses dentaires après nettoyage.

L'invention a pour but de remédier à tout ou partie des inconvénients précités.

L'invention a pour objet une cassette pour prothèses dentaires destinée être utilisée dans un dispositif de nettoyage ou dans un lave-vaisselle, selon la revendication 1.

Avantageusement, la cassette permet de protéger chaque prothèse dentaire de tous contacts extérieurs et de manière individuelle et permet de former un ensemble avec d'autres cassettes dans le dispositif de nettoyage.

Préférentiellement, la première languette de coopération de la première cassette est configurée pour coopérer avec le premier logement de retenue ménagé sur une deuxième cassette destinée à être positionnée sur la première cassette.

Selon une caractéristique de l'invention, la première languette de coopération s'étend dans le prolongement de la hauteur de la cassette. Préférentiellement, la première languette de coopération est ménagée sur la face latérale frontale de la cassette.

Selon une caractéristique de l'invention, le premier logement de retenue est ménagé sur la face frontale de la cassette en regard de la première languette de coopération. Préférentiellement le premier logement de retenue est ménagé à la jointure entre le fond et la face frontale de la cassette.

Avantageusement, le premier logement de retenue est un évidement.

Selon une caractéristique de l'invention, la cassette comprend une deuxième languette de coopération s'étendant dans un plan sensiblement perpendiculaire à celui dans lequel la première languette de coopération s'étend.

Selon une caractéristique de l'invention, la cassette comprend un couvercle agencé en regard du fond et configuré pour fermer le volume interne de la cassette.

Selon une caractéristique de l'invention, la deuxième languette de coopération est positionnée sur le couvercle de la cassette.

Selon une caractéristique de l'invention, la deuxième languette de coopération comprend un orifice dans lequel, lorsque la cassette est fermée par le couvercle, la première languette de coopération passe.

Selon une caractéristique de l'invention, la première languette de coopération de la cassette est configurée pour coopérer avec la deuxième languette de coopération de la cassette et avec un premier logement de retenue d'une deuxième cassette adjacente et préférentiellement agencée sur la cassette.

La coopération entre le premier logement de retenue et la première languette de coopération est réalisée par complémentarité de forme.

Selon une caractéristique, la deuxième languette de coopération est configurée pour coopérer avec un deuxième logement de retenue ménagé sur une autre cassette destinée à être positionnée devant la première cassette.

Selon une caractéristique, le deuxième logement de retenue est ménagé sur le couvercle de la cassette en regard de la deuxième languette de coopération. Avantageusement, le deuxième logement de retenue est un évidement.

Selon une caractéristique de l'invention, la cassette comprend une pluralité de trous d'égouttage. Avantageusement, les trous d'égouttage permettent le passage des liquides de nettoyage et de rinçage et la pénétration des ondes le cas échéant.

Selon une caractéristique de l'invention, la cassette comprend un système de fermeture par pression. Préférentiellement, la première languette de coopération est formée d'un premier pan incliné dans une première direction surmonté d'un deuxième pan incliné dans une deuxième direction opposée à la première direction. Cette forme de languette permet le verrouillage de la languette de coopération par rapport au logement de retenue et par rapport à l'orifice de la deuxième languette de coopération.

Selon l'invention, la cassette comprend un système de maintien de prothèses dentaires. Ce système de maintien permet d'éviter que la prothèse dentaire ne bouge lors de son nettoyage et ne sorte de la cassette et donc d'éviter que les crochets de la prothèse ne se cassent ou ne s'accrochent.

Selon l'invention, le système de maintien est logé dans le volume intérieur de la cassette.

Selon l'invention, le système de maintien comprend un ressort hélicoïdal configuré pour être en position de repos lorsque la cassette est vide et dans une position de compression quand la cassette contient une prothèse dentaire.

Selon une caractéristique, le ressort est fixé au fond de la cassette. Selon une autre caractéristique, le ressort est en outre fixé sur la plaque support.

Selon l'invention, le système de maintien comprend en outre une plaque de support agencé dans le volume interne de la cassette.

Selon l'invention, la cassette comprend une pluralité d'ailettes de support configurées pour maintenir la plaque de support sur le ressort.

Selon une autre caractéristique, chaque ailette de la pluralité s'étend dans un plan sensiblement parallèle au fond de la cassette.

Selon une caractéristique de l'invention, la cassette comprend un organe d'identification par exemple une plaque numérotée. Avantageusement, l'organe d'identification peut être rajouté sur une face de la cassette.

Selon une autre caractéristique, l'organe d'identification se présente sous la forme d'une plaquette-pince configurée pour s'insérer sur une face latérale de la cassette présentant ou non un évidement pour faciliter son insertion.

Selon une caractéristique de l'invention, la cassette comprend au moins un compensateur antichoc ménagé à l'extérieur du volume de la cassette, ce qui permet d'éviter les chocs pendant le nettoyage de la prothèse dentaire et une déformation de la cassette.

Selon une caractéristique, le au moins un compensateur antichoc est une bande, préférentiellement en plastique alimentaire positionnée sur la cassette. Préférentiellement, le plastique alimentaire est par exemple un silicone.

Préférentiellement, la cassette comprend une pluralité de compensateurs antichoc.

L'invention a également pour objet un ensemble de cassettes comprenant au moins deux cassettes selon l'invention.

Selon une caractéristique, la deuxième cassette de l'ensemble est positionnée sur la première cassette de l'ensemble de sorte que la première languette de coopération de la première cassette coopère avec le premier logement de retenue de la deuxième cassette.

Alternativement, la deuxième cassette est positionnée devant la première cassette de l'ensemble, de sorte que la deuxième languette de coopération de la première cassette coopère avec le deuxième logement de retenue de la deuxième cassette.

Selon une caractéristique, l'ensemble comprend une troisième cassette positionnée devant la première cassette de l'ensemble, de sorte que la deuxième languette de coopération de la première cassette coopère avec le deuxième logement de retenue de la troisième cassette et la deuxième cassette de l'ensemble est positionnée sur la première cassette de l'ensemble de sorte que la première languette de coopération de la première cassette coopère avec le premier logement de retenue de la deuxième cassette.

Les cassettes sont réalisées dans un matériau convenant au traitement (nettoyage et rinçage) par ultrasons, et thermo-désinfectant et également au nettoyage dans un lave-vaisselle. Préférentiellement, les cassettes sont réalisées en acier inoxydable de type médical.

L'invention a en outre comme objet un panier de stockage comprenant un volume intérieur conformé pour recevoir une pluralité de cassettes selon l'invention.

Selon une caractéristique de l'invention, le panier de stockage présente au moins une alcôve conformée pour loger une cassette. Préférentiellement, le panier de stockage comprend deux alcôves positionnées en regard l'une de l'autre et chacune ménagée sur une face opposée du panier de stockage, lesdites alcôves sont conformées pour loger chacune une cassette.

Avantageusement, le panier de stockage comprend une structure de type cage permettant d'enfermer dans son volume les cassettes selon l'un quelconque des modes de réalisation de l'invention et décrits ci-après.

Avantageusement, le panier de stockage est réalisé dans un matériau convenant au traitement (nettoyage et rinçage) par ultrasons, et thermo-désinfectant et également au nettoyage dans un lave-vaisselle. Préférentiellement, le panier de stockage est réalisé en acier inoxydable de type médical.

L'invention sera mieux comprise, grâce à la description ci-après, qui se rapporte à des modes de réalisation selon la présente invention, donné à titre d'exemple non limitatif et expliqué avec référence aux dessins schématiques annexés, dans lesquels:
- la figure 1 est une vue schématique d'un dispositif de nettoyage,
- la figure 2 est une vue de face de l'enceinte de nettoyage du dispositif de nettoyage représenté en figure 1,
- la figure 3 est une vue en coupe selon l'axe A-A de l'enceinte de nettoyage représentée en figure 2,
- La figure 4 représente une pluralité de cassettes selon l'invention dans un panier de stockage,
- la figure 5 est une vue en perspective d'une cassette selon un premier mode de réalisation de l'invention,
- la figure 6 est une vue en perspective ouverte de la cassette représentée en figure 5,
- -la figure 7 est une vue de dessus de la cassette représentée en figure 5,
- La figure 8 est une vue de face de la cassette représentée en figure 5,
- La figure 9 est une vue de l'ensemble de cassettes selon l'invention,
- la figure 10 est une vue de détail selon l'encadré B de la figure 9,
- la figure 11 est une vue de détail selon l'encadré C de la figure 9,
- la figure 12 est une vue en perspective de la cassette selon un deuxième mode de réalisation de l'invention,
- la figure 13 est une vue éclatée de la cassette représenté en figure 12,
- les figures 14 et 15 illustrent une première variante de réalisation de l'organe d'identification, applicable à la cassette selon l'un quelconque des modes de réalisation
- les figures 16 et 17 illustrent une deuxième variante de réalisation de l'organe d'identification, applicable à la cassette selon l'un quelconque des modes de réalisation.

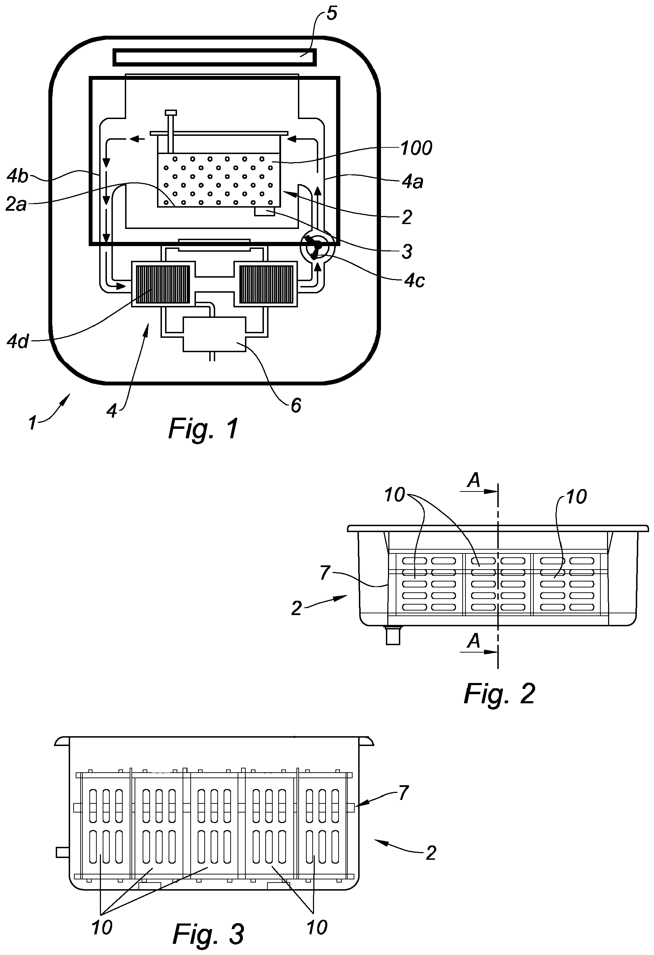

Cette divulgation porte notamment sur un dispositif de nettoyage 1 pour prothèses dentaires (non représentées) comprenant une enceinte de nettoyage 2 comportant une portion de réception 2a d'au moins une prothèse dentaire. L'enceinte de nettoyage 1 est destinée à contenir en état de fonctionnement une solution nettoyante comme illustré en figure 1.

Comme illustré en figure 1, le dispositif de nettoyage 1 comprend en outre un transducteur 3 pour ultrasons positionné au niveau de l'enceinte de nettoyage 2 et configuré pour, en état de fonctionnement, générer des phases successives de compression et de décompression dans la solution liquide de nettoyage et de rinçage 100. Dans l'exemple illustré en figure 1, le transducteur 3 pour ultrasons est positionné au contact de l'enceinte de nettoyage 2, sur la paroi externe du fond de l'enceinte de nettoyage 2. Bien entendu, la position du transducteur n'est pas limitative et pourrait être toute autre : sur les parois internes ou externes de l'enceinte ou au fond de l'enceinte.

Le dispositif de nettoyage 1 comprend un système de séchage 4 par air chaud intégré dans le dispositif de nettoyage 1 et configuré pour coopérer avec l'enceinte de nettoyage 2 comme illustré en figure 1. Le système de séchage 4 est un système de ventilation et/ ou de chauffage par circulation forcée d'air chaud, le système de chauffage comprenant au moins un conduit 4a, 4b débouchant dans l'enceinte de nettoyage 2. Avantageusement, le système de séchage comprend en outre un condensateur 4d, et un ventilateur 4c pour envoyer l'air chaud et un système de pompe à chaleur 6 au niveau de l'enceinte de nettoyage 2.

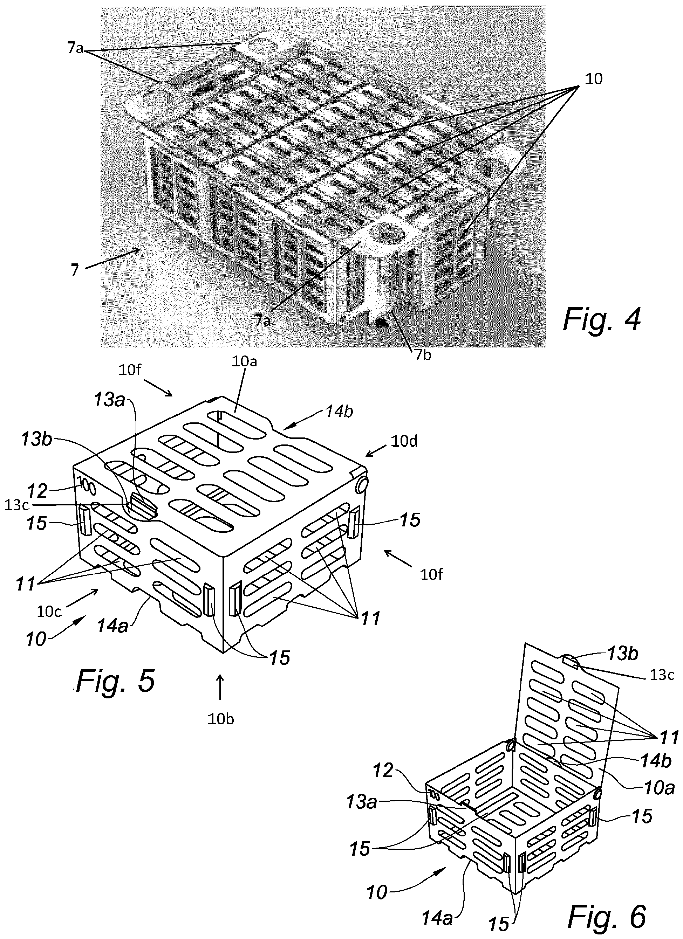

L'enceinte de nettoyage 2 comprend une portion de réception 2a qui est configurée pour coopérer avec au moins une cassette 10 conformée pour loger au
moins une prothèse dentaire, quel que soit le mode de réalisation de ladite cassette représentés aux figures 4 à 17. Préférentiellement, la portion de réception 2a de l'enceinte de nettoyage 2 coopère avec un panier 7 de stockage et de transport, comme illustré aux figures 2 à 4.

En figure 1 est illustré un panier de stockage 7, comprenant un volume intérieur conformé pour recevoir une pluralité de cassettes 10 selon l'invention. Le panier de stockage 7, présente des zones de préhension 7a et des pieds 7b. En outre le panier de stockage 7 présente deux alcôves 7c positionnées en regard l'une de l'autre et chacune ménagée sur une face opposée du panier de stockage 7. Les alcôves 7c sont conformées pour loger chacune une cassette 10. Le panier de stockage 7 comprend une structure de type cage permettant d'enfermer dans son volume des cassettes 10 selon l'un quelconque des modes de réalisation de l'invention et décrits ci-après.

Le panier de stockage 7 est réalisé dans un matériau convenant au traitement (nettoyage et rinçage) par ultrasons, et thermo-désinfectant et également au nettoyage dans un lave-vaisselle. Préférentiellement, le panier de stockage 7 est réalisé en acier inoxydable de type médical.

La cassette 10 selon le premier mode de réalisation va maintenant être décrite en référence aux figures 4 à 8.

La cassette 10 présente un volume interne destiné à recevoir au moins une prothèse dentaire. Comme visible sur les figures 4 à 6, la cassette 10 comprend une pluralité de trous d'égouttage 11. Les trous d'égouttages 11 sont répartis sur toutes les faces 10a, 10b, 10c, 10d, 10f, de la cassette 10 de manière à pouvoir évacuer les différents bains de traitement.

La cassette 10 comprend un couvercle 10a, un fond 10b, une face latérale frontale 10c, une face latérale arrière 10d et deux faces latérales de côté 10f.

Avantageusement, et comme illustré en figure 6 par exemple, la cassette 10 comprend un organe d'identification 12 par exemple une plaque numérotée gravée ou collée ou analogue sur une au moins une des faces 10a, 10b, 10c, 10d, 10f de la cassette 10, préférentiellement sur la face latérale frontale 10c.

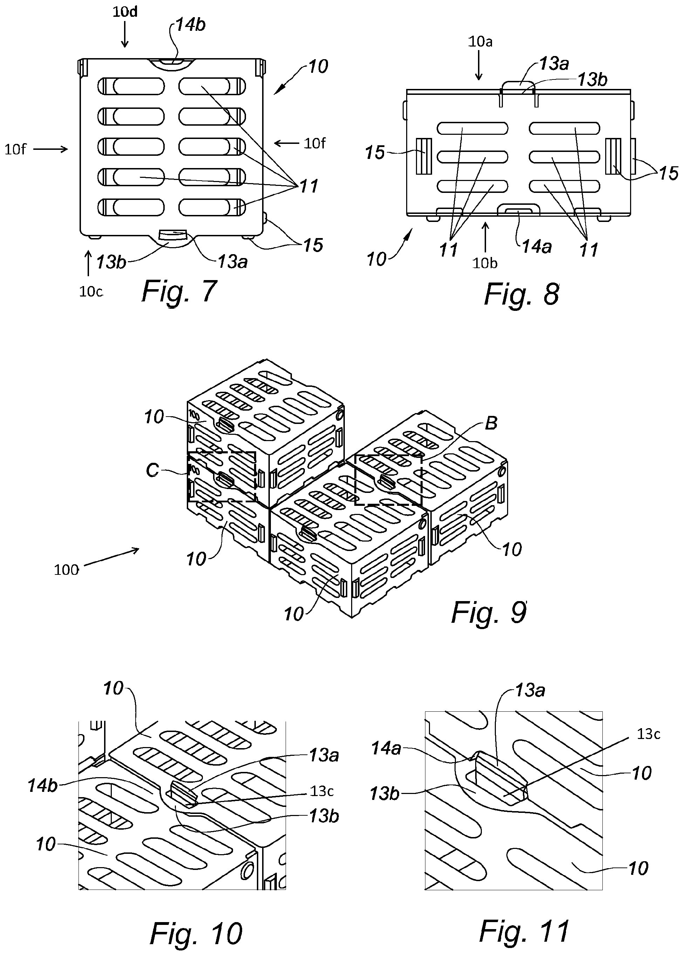

En outre, comme visible aux figures 7 et 8, la cassette 10 comprend une première languette de coopération 13a saillante par rapport au volume de la cassette 10.

La première languette de coopération 13a est configurée pour coopérer avec un premier logement de retenue 14a ménagé sur une autre cassette 10 destinée à être positionnée sur la première cassette 10 comme illustré aux figures 9 et 10.

Plus précisément, la première languette de coopération 13a s'étend dans le prolongement de la hauteur de la cassette 10 et sur la face latérale frontale 10c de la cassette 10.

En outre, comme visible aux figures 9 et 10, la cassette 10 comprend une deuxième languette de coopération 13b saillante par rapport au volume de la cassette 10. Avantageusement, la deuxième languette de coopération 13b s'étend dans un plan sensiblement perpendiculaire à celui dans lequel la première languette de coopération 13a s'étend. La deuxième languette de coopération 13b s'étend sur le couvercle 10a de la cassette 10.

Avantageusement, la deuxième languette de coopération 13b comprend un orifice 13c dans lequel, lorsque la cassette 10 est fermée, la première languette de coopération 13a passe.

Quel que soit le mode de réalisation de la cassette 10, chaque cassette 10 comprend un premier logement de retenue 14a ménagé sur la face frontale 10c de la cassette 10, en regard de la première languette de coopération 13a comme illustré aux figures 5, 6, 12 et 13. En outre, chaque cassette 10 comprend un deuxième logement de retenue 14b ménagé sur le couvercle 10a de la cassette 10, en regard de la deuxième languette de coopération 13b comme illustré aux figures 5, 6, 12 et 13.

Chaque languette de coopération 13a, 13b coopère respectivement avec un logement de retenue 14a, 14b notamment par complémentarité de forme. La deuxième languette de coopération 13b est configurée pour coopérer avec un deuxième logement de retenue 14b ménagé sur une autre cassette 10 destinée à être positionnée devant la première cassette 10 comme visible à la figure 9. Encore plus précisément, le couvercle 10a est monté sur pivot et est destiné à permettre l'accès au volume interne de la cassette 10 lorsqu'elle est ouverte. La deuxième languette de coopération 13b est ménagée sur ledit couvercle 10a.

La cassette 10 comprend une pluralité de compensateurs 15 antichocs ménagés à l'extérieur du volume de la cassette 10, ce qui permet d'éviter les chocs pendant le nettoyage de la prothèse dentaire et une déformation de la cassette 10.

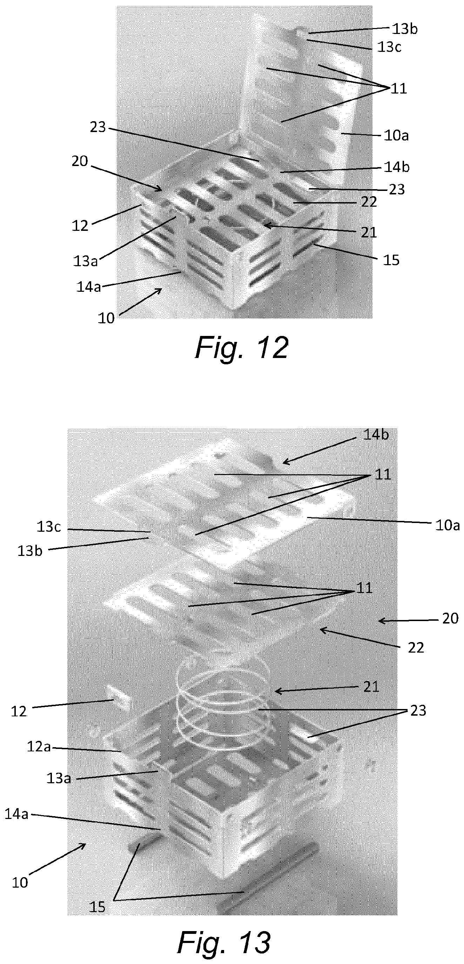

La cassette 10 selon l'invention va maintenant être décrite en référence aux figures 12 et 13. La cassette 10 selon l'invention diffère de la cassette 10 selon le premier mode de réalisation seulement en ce que la cassette 10 comprend un système de maintien 20 de prothèses dentaires. Comme on peut le voir en figure 12 notamment le système de maintien 20 est logé dans le volume intérieur de la cassette 10.

Le système de maintien 20 comprend un ressort hélicoïdal 21 configuré pour être en position de repos lorsque la cassette 10 est vide et dans une position de compression quand la cassette 10 contient une prothèse dentaire.

Le système de maintien 20 comprend en outre une plaque de support 22 agencé dans le volume interne de la casse 10. La cassette 10 comprend une pluralité d'ailettes 23 de support configurées pour maintenir la plaque de support 22 sur le ressort 21. Avantageusement, chaque ailette 23 de la pluralité s'étend dans un plan sensiblement parallèle au fond de la cassette 10. Comme illustré aux figures 11 et 12, la cassette 10 comprend deux ailettes 23 agencés sur une première face latérale et deux autres ailettes 23 (non visibles) agencées sur une deuxième face latérale opposée à la première la face opposée. Bien entendu, le nombre d'ailettes et leur répartition sur les faces latérales de la cassette 10 ne sont pas limités à l'exemple illustré.

Lorsque le ressort est en position de compression, ce dernier force le support 22 recevant la prothèse dentaire à aller vers le couvercle 10a de la cassette 10, de sorte que le support 22 vient en appui contre les ailettes 23.

Ce système de maintien 20 permet d'éviter que la prothèse dentaire ne bouge lors de son nettoyage et ne sorte de la cassette 10 et donc d'éviter que les crochets de la prothèse ne se cassent ou ne s'accrochent.

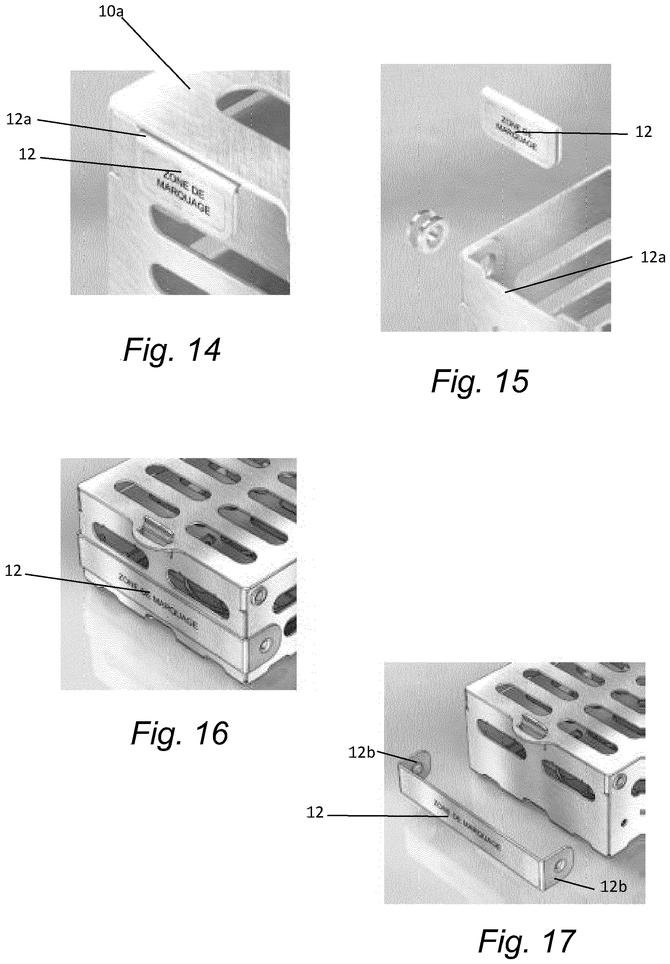

Avantageusement et comme illustré en figure 15, la cassette 10 selon un mode de réalisation comprend une pluralité de compensateurs 15 antichocs positionnés sur l'extérieur de la cassette 10 et notamment sur l'extérieur du fond de la cassette 10. L'ensemble des autres caractéristiques relatives à la structure, à la fermeture/ouverture de la cassette 10 et à la coopération avec des cassettes adjacentes sont les mêmes que celles de la cassette 10 selon le premier mode de réalisation.

Les figures 14 et 15 illustrent une première variante de réalisation de l'organe d'identification 12 de la cassette 10 quel que soit le mode de réalisation de la cassette 10. Dans cette première variante de réalisation, on réalise un évidement 12a sur une des faces latérales de la cassette 10, puis on vient clipper ou insérer l'organe d'identification 12 dans cet évidement 12a. Avantageusement, dans cette variante de réalisation, l'organe d'identification 12 se présente sous la forme d'une plaquette-pince. Bien entendu, l'organe d'identification 12 peut également s'insérer sur une face latérale de la cassette sans l'évidement.

Les figures 16 et 17 illustrent une deuxième variante de réalisation de l'organe d'identification 12 de la cassette 10 quel que soit le mode de réalisation de la cassette 10. Dans cette deuxième variante de réalisation, l'organe d'identification 12 se présente sous la forme d'une plaque présentant deux oreilles 12b. L'organe d'identification 12 est conformé pour venir se plaquer contre la face latérale frontale 10c de la cassette 10, les oreilles 12b de l'organe d'identification venant chacune contre une portion d'une face latérale de côté 10f de la cassette 10. Avantageusement, les oreilles 12b sont fixées sur la cassette 10 pour maintenir l'organe d'identification sur la cassette 10.

## Revendications

1. Cassette (10) pour prothèses dentaires destinée être utilisée dans un dispositif de nettoyage ou dans un lave-vaisselle, ladite cassette (10) présentant au moins un fond (10b), une face latérale frontale (10c), une face latérale arrière (10d) et deux faces latérales de côté (10f), lesdites faces (10c, 10d, 10f) et le fond (10b) délimitant un volume interne de la cassette (10) configuré pour loger une prothèse
dentaire, ladite cassette (10) comprenant :
- au moins une première languette de coopération (13a) saillante par rapport au volume interne, ladite première languette de coopération (13a) étant configurée pour coopérer avec un premier logement de retenue (14a) ménagé sur une autre cassette (10) adjacente,
- au moins un premier logement de retenue (14a) configuré pour coopérer avec une première languette de coopération (13a) ménagé sur une autre cassette (10) adjacente,
- un système de maintien (20) de prothèses dentaires logé dans le volume intérieur de la cassette (10), le système de maintien (20) comprenant le système de maintien (20) comprenant en outre une plaque de support (22) agencé dans le volume interne de la cassette (10), **caractérisée en ce que** le système de maintien comprend un ressort (21) hélicoïdal configuré pour être en position de repos lorsque la cassette (10) est vide et dans une position de compression quand la cassette (10) contient une prothèse dentaire et une pluralité d' ailettes de support (23) configurées pour maintenir la plaque de support (22) sur le ressort (21)

2. Cassette selon la revendication 1, dans laquelle la première languette de coopération (13a) s'étend dans le prolongement de la hauteur de la cassette (10) et préférentiellement, la première languette de coopération (13a) est ménagée sur la face latérale frontale (10c) de la cassette (10).

3. Cassette selon l'une quelconque des revendications 1 ou 2, dans laquelle le premier logement de retenue (14a) est ménagé sur la face frontale (10c) de la cassette (10) en regard de la première languette de coopération (13a).

4. Cassette selon l'une quelconque des revendications 1 à 3, comprenant un couvercle (10a) agencé en regard du fond (10b) et configuré pour fermer le volume interne de la cassette (10).

5. Cassette selon l'une quelconque des revendications 1 à 4, comprenant une deuxième languette de coopération (13b) s'étendant dans un plan sensiblement perpendiculaire à celui dans lequel la première languette de coopération (13a) s'étend.

6. Cassette selon la revendication 5 prise en combinaison avec la revendication 4, dans laquelle la deuxième languette de coopération (13b) est positionnée sur le couvercle (10a) de la cassette (10).

7. Cassette selon l'une quelconque des revendications 5 ou 6, dans laquelle la deuxième languette de coopération (13b) comprend un orifice dans lequel, lorsque la cassette (10) est fermée par le couvercle (10a), la première languette de coopération (13a) passe.

8. Cassette selon l'une quelconque des revendications 5 à 7, dans laquelle la première languette de coopération (13a) de la cassette (10) est configurée pour coopérer avec la deuxième languette de coopération (13b) de la cassette (10) et avec un premier logement de retenue (14a) d'une deuxième cassette (10) adjacente.

9. Cassette selon l'une quelconque des revendications 1 à 8, comprenant un organe d'identification (12) par exemple une plaque numérotée.

10. Cassette selon l'une quelconque des revendications 1 à 9, comprenant au moins un compensateur antichoc (15) ménagé à l'extérieur du volume de la cassette (10).

11. Cassette selon l'une quelconque des revendications 1 à 10, comprenant une pluralité de trous d'égouttage (11).

12. Cassette selon l'une quelconque des revendications 1 à 11, comprenant un système de fermeture par pression.

13. Cassette selon l'une quelconque des revendications 1 à 12, **caractérisée en ce qu'**elle est réalisée dans un matériau convenant au traitement par ultrasons, et thermo-désinfectant et également au nettoyage dans un lave-vaisselle.

14. Ensemble (100) de cassettes comprenant au moins une première cassette (10) et une deuxième cassette (10) selon l'une quelconque des revendications 1 à 13.

## Patentansprüche

1. Kassette (10) für Zahnprothesen, die dazu bestimmt ist, in einer Reinigungsvorrichtung oder in einem Geschirrspüler verwendet zu werden, wobei die Kassette (10) mindestens einen Boden (10b), eine vordere seitliche Fläche (10c), eine hintere seitliche Fläche (10d) und zwei seitliche Seitenflächen (10f) aufweist, wobei die Flächen (10c, 10d, 10f) und der Boden (10b) einen Innenraum der Kassette (10) bilden, der ausgelegt ist, um eine Zahnprothese aufzunehmen, wobei die Kassette (10) Folgendes umfasst:
- mindestens eine erste Zusammenwirkungslasche (13a), die bezüglich des Innenraums vorragt, wobei die erste Zusammenwirkungslasche (13a) ausgelegt ist, um mit einer ersten Rückhalteaufnahme (14a), die auf einer anderen benachbarten Kassette (10) eingerichtet ist, zusammenzuwirken,
- mindestens eine erste Rückhalteaufnahme (14a), die ausgelegt ist, um mit einer ersten Zusammenwirkungslasche (13a), die auf einer anderen benachbarten Kassette (10) eingerichtet ist, zusammenzuwirken,
- ein Haltesystem (20) von Zahnprothesen, das in dem Innenraum der Kassette (10) aufgenommen wird, wobei das Haltesystem (20) außerdem eine Stützplatte (22) umfasst, die in dem Innenraum der Kassette (10) eingerichtet ist, **dadurch gekennzeichnet, dass** das Haltesystem eine Schraubenfeder (21) umfasst, die ausgelegt ist, um in Ruheposition, wenn die Kassette (10) leer ist, und in einer Kompressionsposition zu sein, wenn die Kassette (10) eine Zahnprothese enthält, und eine Vielzahl von Stützrippen (23), die ausgelegt sind, um die Stützplatte (22) auf der Feder (21) zu halten.

2. Kassette nach Anspruch 1, wobei sich die erste Zusammenwirkungslasche (13a) in der Verlängerung der Höhe der Kassette (10) erstreckt und die erste Zusammenwirkungslasche (13a) bevorzugt auf der vorderen seitlichen Fläche (10c) der Kassette (10) eingerichtet ist.

3. Kassette nach einem der Ansprüche 1 oder 2, wobei die erste Rückhalteaufnahme (14a) auf der Vorderfläche (10c) der Kassette (10) gegenüber der ersten Zusammenwirkungslasche (13a) eingerichtet ist.

4. Kassette nach einem der Ansprüche 1 bis 3, die einen Deckel (10a) umfasst, der gegenüber dem Boden (10b) eingerichtet und ausgelegt ist, um den Innenraum der Kassette (10) zu schließen.

5. Kassette nach einem der Ansprüche 1 bis 4, die eine zweite Zusammenwirkungslasche (13b) umfasst, die sich in einer Ebene im Wesentlichen senkrecht zu der, in der sich die erste Zusammenwirkungslasche (13a) erstreckt, erstreckt.

6. Kassette nach Anspruch 5, in Kombination mit Anspruch 4 genommen, wobei die zweite Zusammenwirkungslasche (13b) auf dem Deckel (10a) der Kassette (10) positioniert ist.

7. Kassette nach einem der Ansprüche 5 oder 6, wobei die zweite Zusammenwirkungslasche (13b) eine Öffnung umfasst, in der, wenn die Kassette (10) von dem Deckel (10a) verschlossen wird, die erste Zusammenwirkungslasche (13a) durchgeht.

8. Kassette nach einem der Ansprüche 5 bis 7, wobei die erste Zusammenwirkungslasche (13a) der Kassette (10) ausgelegt ist, um mit der zweiten Zusammenwirkungslasche (13b) der Kassette (10) und mit einer ersten Rückhalteaufnahme (14a) einer zweiten benachbarten Kassette (10) zusammenzuwirken.

9. Kassette nach einem der Ansprüche 1 bis 8, die ein Identifikationselement (12), zum Beispiel eine nummerierte Platte, umfasst.

10. Kassette nach einem der Ansprüche 1 bis 9, die mindestens einen Stoßschutzkompensator (15) umfasst, der außerhalb des Raums der Kassette (10) eingerichtet ist.

11. Kassette nach einem der Ansprüche 1 bis 10, die eine Vielzahl von Abtropflöchern (11) umfasst.

12. Kassette nach einem der Ansprüche 1 bis 11, die ein Druckverschlusssystem umfasst.

13. Kassette nach einem der Ansprüche 1 bis 12, **dadurch gekennzeichnet, dass** sie aus einem Material hergestellt ist, das für Ultraschallbehandlung und Wärmedesinfektionsmittel und auch zum Reinigen in einem Geschirrspüler geeignet ist.

14. Einheit (100) von Kassetten, die mindestens eine erste Kassette (10) und eine zweite Kassette (10) nach einem der Ansprüche 1 bis 13 umfasst.

## Claims

1. A cassette (10) for dental prostheses intended to be used in a cleaning device or in a dishwasher, said cassette (10) having at least one bottom (10b), one front lateral face (10c), one rear lateral face (10d) and two side lateral faces (10f), said faces (10c, 10d, 10f) and the bottom (10b) delimiting an internal volume of the cassette (10) configured to house a dental prosthesis, said cassette (10) comprising:
- at least one first cooperating tab (13a) protruding relative to the internal volume, said first cooperating tab (13a) being configured to cooperate with a first retaining housing (14a) formed on another adjacent cassette (10),
- at least one first retaining housing (14a) configured to cooperate with a first cooperating tab (13a) formed on another adjacent cassette (10),
- a dental prostheses holding system (20) housed in the interior volume of the cassette (10), the holding system (20) comprising a support plate (22) arranged in the internal volume of the cassette (10), **characterized in that** the holding system comprises a helical spring (21) configured to be in the rest position when the cassette (10) is empty and in the compression position when the cassette (10) contains a dental prosthesis and a plurality of support fins (23) configured to maintain the support plate (22) on the spring (21).

2. The cassette according to claim 1, wherein the first cooperating tab (13a) extends in the extension of the height of the cassette (10) and preferably, the first cooperating tab (13a) is formed on the front lateral face (10c) of the cassette (10).

3. The cassette according to any one of claims 1 or 2, wherein the first retaining housing (14a) is formed on the front face (10c) of the cassette (10) facing the first cooperating tab (13a).

4. The cassette according to any one of claims 1 to 3, comprising a cover (10a) arranged facing the bottom (10b) and configured to close the internal volume of the cassette (10).

5. The cassette according to any one of claims 1 to 4, comprising a second cooperating tab (13b) extending in a plane substantially perpendicular to that in which extends the first cooperating tab (13a).

6. The cassette according to claim 5 taken in combination with claim 4, wherein the second cooperating tab (13b) is positioned on the cover (10a) of the cassette (10).

7. The cassette according to any one of claims 5 or 6, wherein the second cooperating tab (13b) comprises an orifice through which passes the first cooperating tab (13a) when the cassette (10) is closed by the cover (10a).

8. The cassette according to any one of claims 5 to 7, wherein the first cooperating tab (13a) of the cassette (10) is configured to cooperate with the second cooperating tab (13b) of the cassette (10) and with a first retaining housing (14a) of a second adjacent cassette (10).

9. The cassette according to any one of claims 1 to 8, comprising an identification member (12) for example a numbered plate.

10. The cassette according to any one of claims 1 to 9, comprising at least one shock-absorbing compensator (15) formed outside the volume of the cassette (10).

11. The cassette according to any one of claims 1 to 10, comprising a plurality of draining holes (11).

12. The cassette according to any one of claims 1 to 11, comprising a pressure closure system.

13. The cassette according to any one of claims 1 to 12, **characterized in that** it is made of a material suitable for ultrasonic and thermo-disinfectant treatment and also for cleaning in a dishwasher.

14. A set (100) of cassettes comprising at least one first cassette (10) and one second cassette (10) according to any one of claims 1 to 13.
